# EUROPEAN PATENT APPLICATION

(11) **EP 4 042 971 A1**
(43) Date of publication of application: **17.08.2022**
(21) Application number: 20874132.2
(22) Date of filing: 27.09.2020
(51) Int. Cl.: A61F 2/24

(54) **HEART VALVE STENT AND PROSTHESIS THEREOF**

(30) Priority: 12.10.2019 CN 201910970095
(71) Applicant: Shanghai Microport Cardioflow Medtech Co., Ltd., Shanghai 201203 (CN)
(72) Inventor: TAN, Jian, Shanghai 201203 (CN); LIU, Ming, Shanghai 201203 (CN); JI, Lijun, Shanghai 201203 (CN); CHEN, Guoming, Shanghai 201203 (CN); LI, Yu, Shanghai 201203 (CN)
(74) Representative: Dauncey, Mark Peter
(86) International application number: PCT/CN2020/118028
(87) International publication number: WO 2021/068772

(57) **Abstract**

The present invention discloses a valve stent and a prosthesis thereof. The valve stent includes a frame including a plurality of mesh cells connected with each other, a plurality of end mesh cells are provided at two ends of the frame, at least one of the end mesh cells is a first end mesh cell, all of the end mesh cells at an end where the first end mesh cell is located are arranged continuously and completely in a circumferential direction, and a height of a projection of the first end mesh cell in an axial direction is lower than a height of a projection of rest of the end mesh cells at the end where the first end mesh cell is located. The valve stent and the prosthesis thereof provided by the present invention can reduce the damage to the conduction system and also reduce the conduction block, thereby improving the safety of the operation and further the quality of life of the patient.

## Description

### TECHNICAL FIELD

The present invention relates to an interventional medical prosthesis, more specifically, to a valve stent and a prosthesis thereof.

### BACKGROUND

There are four valves in the human heart, including the aortic valve, the pulmonary valve, the mitral valve and the tricuspid valve. They all play the role of one-way valves. In the blood circulation, with the rhythmic contraction and relaxation of the heart, the heart valves also open and close rhythmically, so that the blood can pass through the valve smoothly and can be prevented from regurgitation. As a result, the blood can flow circularly in a certain direction in human body so as to maintain the normal function of the circulatory system. When the heart valve is inflamed, it will cause structural damage, fibrosis, adhesion, shortening, myxomatous degeneration, ischemic necrosis, calcium precipitation. In addition, congenital developmental malformations will also cause valve disease, affecting normal blood circulation, medically known as valvular heart disease. The incidence of aortic stenosis (AS) due to aortic valve degeneration in the elderly over 65 years old is up to 10%, and the most common type is calcific aortic stenosis (CAS).

Interventional heart valve surgical operation is a medical technology that has developed rapidly in recent years, which aims to implant, through the apex or blood vessel, the valve prosthesis to the position where the native valve exists to replace it while causing microtrauma, and finally achieve the purpose of treating the patient. The operation is characterized in less trauma, fast recovery and low risk, and is especially suitable for elderly patients who suffer from valvular heart disease. Transcatheter aortic valve implantation (TAVI) is a kind of heart valve interventional therapy for aortic valve disease, which is mainly used for the treatment of valvular heart diseases such as aortic stenosis and aortic insufficiency.

### SUMMARY OF THE INVENTION

### Technical Problem

At present, there are two kinds of aortic valve stent systems that have been put into clinical application. One is a valve stent whose main structure is made of rigid materials and needs to be released by balloon expansion. The other is a valve stent whose main structure is formed of memory metal materials and requires self-expansion for in vivo release. Valve stents that rely on balloon-expansion release are implanted through the apex, while self-expanding valve stents are implanted via peripheral vascular access. The implantation via peripheral vascular access is characterized in less trauma, faster recovery, and thus it is more popular. The self-expanding release has less impact on hemodynamics, when compared with the balloon-expansion release. In addition, self-expanding stents have the advantages of being adjustable and retrievable. However, the most advantage of valve stents that rely on balloon-expandable release is that they cause less atrioventricular block. On the contrary, the proportion of patients implanted with self-expanding valve stents, who further require an implantation of a permanent cardiac pacemaker is significantly higher than the proportion of patients implanted with the valve stents that rely on balloon-expansion release.

Clinical studies have shown that direct compression of the conduction system by aortic valve stents is the most critical factor for new conduction disorders after TAVI. Compared with the self-expandable valve stent implanted with a segment thereof having a length of 4-6 mm in the left ventricle, the conduction disorders are less likely to occur in the valve stent that relies on balloon-expansion release and has a segment with a very small length that is implanted in the outflow tract of the left ventricle. In fact, for the self-expandable valve stent, the deeper it is implanted in the left ventricle, the more likely the conduction block occurs after the operation. However, the high blood flow pressure in the left ventricle has a large impact on the stent and the membrane thereon during the impulse of the left ventricle, the stent therefore is required to be firmly anchored and have a sufficient radial support. Otherwise, it is easy to cause movement and collapse of the stent as well as paravalvular leakage, and even loss of function. At present, in order to ensure that the aortic valve stent has sufficient radial support, a dense and circumferentially complete mesh cell arrangement of the inflow tract is generally adopted.

The existing self-expandable aortic valve stent system is likely to damage the conduction system, thereby causing conduction block, serious complications and even death. Therefore, for the self-expandable aortic valve stent system, how to reduce the risk of conduction block is an urgent problem to be solved, which is very important to promote the technology and improve the quality of life of patients.

### Solution to Problem

### Technical Solution

An object of the present invention is to provide a valve stent and a prosthesis thereof, which can reduce the damage to the conduction system and also reduce the conduction block, thereby improving the safety of the operation and further the quality of life of the patients.

In order to solve the above problem, the present invention provides a valve stent, comprising a frame comprising a plurality of mesh cells connected with each other, wherein a plurality of end mesh cells are provided at two ends of the frame, at least one of the end mesh cells is a first end mesh cell, all of the end mesh cells at an end where the first end mesh cell is located are arranged continuously and completely in a circumferential direction, and a height of a projection of the first end mesh cell in an axial direction is lower than a height of a projection of rest of the end mesh cells at the end where the first end mesh cell is located.

Preferably, an end of the first end mesh cell is inward curled toward an axis of the valve stent to form an inward curled mesh cell

Preferably, in a plane defined by a profile line of the inward curled mesh cell and the axis of the valve stent, an inward curling angle γ satisfies 90°≤γ≤270°.

Preferably, a vertical distance L from a center of a curled portion of the inward curled mesh cell to the profile line is greater than or equal to an arc radius R of the curled portion, wherein the arc radius R is constant or variable.

Preferably, an end of the first end mesh cell is inward bent or concave toward an axis of the valve stent to form an inward inward bent mesh cell or an inward concave mesh cell, and the inward bent mesh cell has a bent portion having a profile line in a form of an arc, a straight line, a broken line, or a combination thereof.

Preferably, in a plane defined by a profile line of the inward bent mesh cell or of the inward concave mesh cell and the axis of the valve stent, an inward concaving angle α and an inward bending angle β satisfy 10°≤α/β≤80°.

Preferably, the first end mesh cell is quadrilateral with four sides formed by rods, and two adjacent ones of the rods forming an end of the first end mesh cell is shorter than other two of the rods

Preferably, a height difference H in the axial direction between the projection of the first end mesh cell and the projection of the rest of the end mesh cells at the end where the first end mesh cell is located satisfies 0mm<H:≤12mm.

Preferably, height difference H satisfies 2≤H≤4mm.

Preferably, an angle θ resulting from the circumferential arrangement of the first end mesh cell satisfies 20°≤θ≤120°.

In order to solve the above problem, the present invention also provides a valve prosthesis, comprising the above valve stent and a membrane, wherein the membrane is arranged in the frame of the valve stent.

### Beneficial Effects of Invention

### Beneficial Effect

Compared with the prior art, the present invention has the following beneficial effects:
In the valve stent and its prosthesis provided by the present invention, the height of projection of at least one first end mesh cell in the axial direction is lower than the height of projection of the other end mesh cells at the end where the first end mesh cell is located. When in use, the first end mesh cell with the shorter axial projection height is in positional correspondence to the conduction bundles of the native valve annulus, which avoids the contact between the end of the stent valve and the conduction bundles, and reduces the risk of conduction block. Compared with the traditional stent with a gap in the circumferential direction, the valve stent and its prosthesis according to the present invention reduces the risk of conduction block while ensuring a better radial support.

### BRIEF DESCRIPTION OF THE DRAWINGS

Figs. 1a, 1b, and 1c are schematic diagrams of the overall structure of three existing valve stents with different shapes;
Figs. 2a and 2b are structural schematic diagrams showing that the end mesh cell of the valve stent is inward curled to form an arc according to an embodiment of the present invention;
Fig. 2c is a schematic diagrams showing the end mesh cell of the valve stent is inward curled to form an arc which causes a height difference in the axial direction according to an embodiment of the present invention;
Fig. 3a is a front view of the arc formed by inward curling the end mesh cell of the valve stent according to an embodiment of the present invention;
Fig. 3b is a side view of the arc formed by inward curling the end mesh cell of the valve stent according to an embodiment of the present invention;
Fig. 3c is a side view of the arc formed by inward curling the end mesh cell of the valve stent according to an embodiment of the present invention;
Fig. 3d is a schematic diagram showing a curling angle of the arc formed by inward curling the end mesh cell of the valve stent according to an embodiment of the present invention;
Fig. 4a is a schematic diagram showing that the first end mesh cell of the valve stent is inward bent to form an arc according to an embodiment of the present invention;
Fig. 4b is a schematic diagram showing that the first end mesh cell of the valve stent is inward bent to form a broken line according to an embodiment of the present invention;
Fig. 4c is a schematic diagram showing that the first end mesh cell of the valve stent is inward bent to form a straight line according to an embodiment of the present invention;
Fig. 4d is a schematic diagram showing an inward concaving angle resulting from the inward concaving the first end mesh cell of the valve stent according to an embodiment of the present invention;
Fig. 4e is a schematic diagram showing an inward bending angle resulting from the inward bending the first end mesh cell of the valve stent according to an embodiment of the present invention;
Fig. 5a is a front view of a valve stent including a shorter first end mesh cell according to an embodiment of the present invention;
Fig. 5b is a side view of a valve stent including a shorter first end mesh cell according to an embodiment of the present invention;
Fig. 6 is a schematic diagram of the arrangement of the first end mesh cell of the valve stent in the circumferential direction according to an embodiment of the present invention;
Fig. 7a is a schematic diagram of a valve prosthesis implanted in the heart according to an embodiment of the application;
Fig. 7b is a partial enlarged view of part A of Fig. 7a;
Fig. 8 schematically illustrates a test on the support performance of the valve prosthesis according to an embodiment of the application.

### DETAILED DESCRIPTION

The present invention will be described in greater detail below with reference to the accompanying drawings and specific embodiments.

In the following description, numerous specific details are set forth in order to provide a thorough understanding of the present invention. However, it will be apparent to one of ordinary skill in the art that the present invention may be practiced without these specific details. Therefore, the specific details set forth are merely exemplary, and may vary from the spirit and scope of the invention and still be considered to be within the spirit and scope of the present invention.

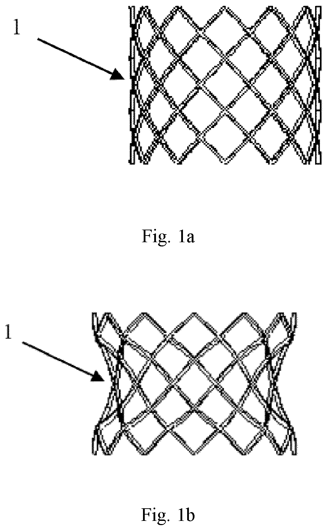

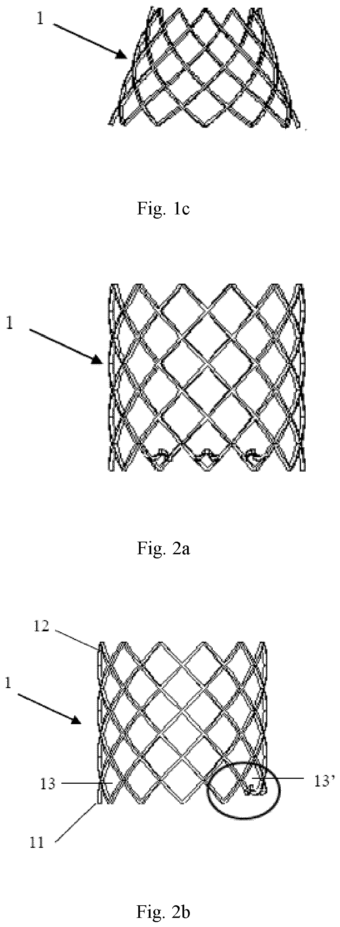

The valve stent proposed in this embodiment can be used for the aortic valve, mitral valve, tricuspid valve or pulmonary valve stent system. In addition to the self-expanding stent, it can also be applied to other types of stents, such as stents with balloon expansion, mechanical expansion. The valve prosthesis proposed in the present invention includes a stent and a membrane. The stent includes a frame. Correspondingly, the membrane can also be radially expanded and compressed, and is located in the frame. Figs 1a, 1b and 1c show three different shapes of frames for valve prostheses. Each frame has a profile presenting a cylindrical shape or a shape similar to a cylinder. The profile of the frame in Fig. 1a has a cylindrical shape, the profile of the frame in Fig. 1b has an hourglass shape, and the profile of the frame in Fig. 1c has a cone shape. Other frame structures similar to cylinders are also possible, which are not limited here. The frame has mash cells and can be compressed into a cylindrical lumen of a tube.

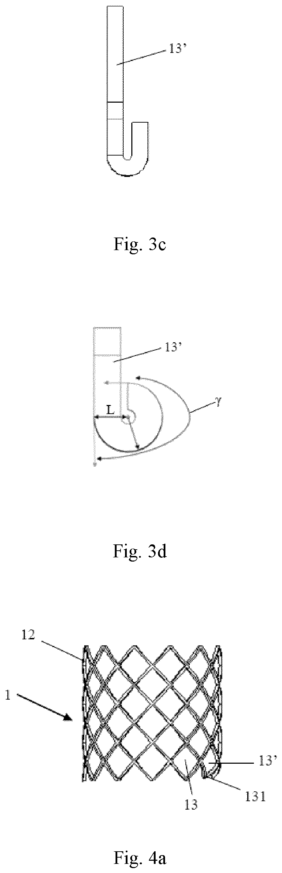

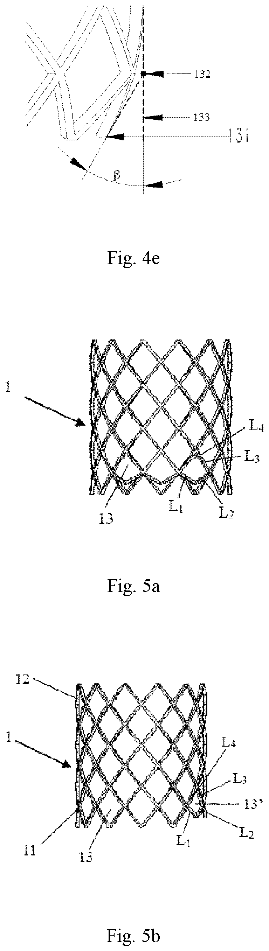

Please refer to Figs. 2a-2c, 4a-4c, 5a and 5b, this embodiment provides a valve stent, including a frame 1. The frame 1 has an inflow tract 11 and an outflow tract 12 in the axial direction. The outflow tract 12 is located downstream of the inflow tract 11 in the direction of blood flow. The inflow tract 11 allows the blood to flow into the valve stent when the valve prosthesis works, and the outflow tract 12 allows the blood to flow out of the valve stent when the valve prosthesis works. The frame 1 consists of a plurality of mesh cells connected with each other. The mesh cells generally have rhombus shapes, and can also be in other suitable closed shapes, such as pentagons, hexagons, etc. The mesh cells include edge rods and nodes. The edge rods are rods forming the mesh cells. The nodes are join points of at least two edge rods. Both ends of the frame 1 have a plurality of end mesh cells 13. At least one of the end mesh cells 13 is a first end mesh cell 13'. All the end mesh cells at the end where the first end mesh cell is located are arranged continuously and completely in the circumferential direction. That is, all the end mesh cells 13 at the end where the first end mesh cell 13' is located are arranged continuously in the circumferential direction without any missing of mesh cells. As a result, the radial support can be ensured. As shown in Figs. 2a-2c and 4a-4c, the end 131 of the first end mesh cell 13' is curled or bent towards the axis of the stent, so that the height of the projection of the first end mesh cell 13' in the axial direction is lower than the height of the projections of the other end mesh cells at the end where the first end mesh cell is located. Optionally, as shown in Figs. 5a and 5b, the two edge rods of each first end mesh cell 13' are shortened, so that the height of the projections of the first end mesh cells 13' in the axial direction is lower than the height of the projections of the other end mesh cells at the end where the first end mesh cell is located. The axis of the stent is a straight line where the central axis of the frame 1 is located. The direction along the axis of the stent is the axial direction. The height of the projection in the axial direction is the height of the projection onto the plane where the axis of the stent is located.

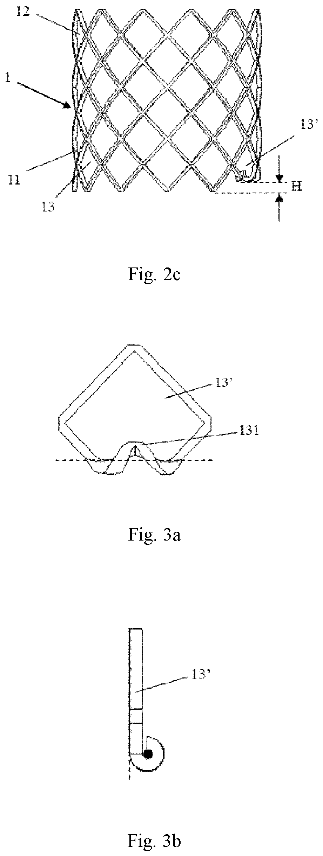

In a specific embodiment, as shown in Figs. 2a-2c and 4a-4c, the end 131 of the first end mesh cell 13' is inward curled, bent or concave towards the axis of the stent to form an inward curled mesh cell, an inward bent mesh cell or an inward concave mesh cell. The inward curled mesh cell, the inward bent mesh cell or the inward concave mesh cell has a resulting deformation portion having a profile in the form of an arc, straight line, broken line or combinations thereof. The end portion with the inward curled mesh cell, the inward bent mesh cell or the inward concave mesh cell is characterized in gathering towards the axis of the stent. Optionally, two edge rods of each first end mesh cell 13' are shortened, so that a height difference occurs in the axial direction between the first end mesh cells 13' having the two shortened edge rods and the other end mesh cells at the end where the first end mesh cells are located. Referring to Figs. 5a and 5b, the mesh cells are embodied as rhombus mesh cells as an example, two adjacent rods L₁, L₂ which forms the end node in each first end mesh cells 13' are shorter than the other two rods L₃, L₄, which enables shorter end mesh cells. The four rods of the other rhombus mesh cells are same in length as the rods L₃, L₄ of the shorter end mesh cells. As a result, a height difference occurs in the axial direction between the shorter first end mesh cells 13' and the other end mesh cells at the end where the first end mesh cells are located. Since the shorter first end mesh cells 13' are also closed mesh cells, the radial support can be ensured. As shown in Fig. 2c, a height difference H occurs in the axial direction between the first end mesh cells 13' and the other end mesh cells at the end where the first end cells are located through inward curling, bending, concaving the ends of the first end mesh cells 13', or by shortening the rods of the first end mesh cells 13'.

Correspondingly, the end 131 of the first end mesh cell 13' in this embodiment refers to the tip of the mesh cell at the end of the profile of the frame. In this embodiment, a generatrix is defined as a moving line whose motion generates a surface; a surface line is defined as a line at any location of the surface; a profile line is defined as a line of the profile on a projective plane..

In one embodiment, please refer to Figs. 2a-2c and 3a-3d, some or all of the end mesh cells 13 at the same end are inward curled towards the axis of the stent to have an arc profile, so as to form an inward curled mesh cells. The inward curling refers to a curling with a certain radius around an axis perpendicular to a plane where the profile line and the axis of the stent are located. The tip of the first end mesh cells 13' is curled toward the axis of the stent as shown in Fig. 3a, or only the inward curled portion forms an arc profile as shown in Fig. 3b, which is similar to the ruffle of a sheet metal. The shape of its inward curled tail end is not limited here, and can be shaped according to needs. In some embodiments, the inward curled portion of the inward curled mesh cell may be formed by arcs with constant or variable radii. The vertical distance from the center of the inward curled portion to the profile line is L, the arc radius is R, then L≥R, where the radius R may be constant or variable. Please refer to Figs. 3a, 3b, 3c and 3d, wherein the dotted line in Fig. 3a represents the axis where the center of the inward curled portion is located, and the dotted line in Fig. 3b is the profile line of the inward curled mesh cell. In the plane defined by the profile line and the axis of the frame 1, the included angle formed by the profile line and the tangent to the end of the arc is an inward curling angle γ (i.e., the rotation angle formed from the start point to the end point of the inward curled portion), as shown in Fig. 3d, where 0.1°≤γ≤360°; preferably, 31°≤γ≤270°, which allows a convenient production; more preferably, 90°≤γ≤270°, in this case, the inward curled portion has concentrated visualization areas which can be observed by the operator so as to ensure accurate positioning, precise fine-tuning or recovery of the valve stent. Please refer to Fig. 2c, the distance H in the axial direction between the projection of the inward curled mesh cell and the projections of the other mesh cells at the end where the inward curled mesh cell is located satisfies 0mm<H≤12mm, preferably, 1≤H≤6mm, more preferably, 2≤H≤4mm.

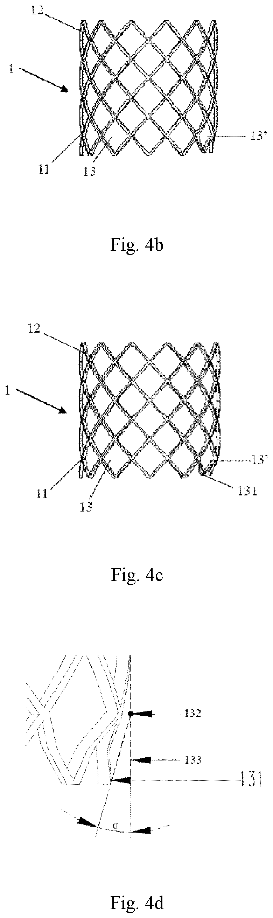

In another embodiment, the end 131 of at least one of the end mesh cells 13 is inward bent or concave towards the axis of the stent, to form an inward bent mesh cell or an inward concave mesh cell. The inward bending allows that the first end mesh cell 13' is bent at a certain portion on the edge thereof toward the axis of the stent, and the tip of the first end mesh cell 13' moves toward the axis of the stent. The inward concave allows that the first end mesh cell 13' is concave at a certain portion on the edge thereof toward the axis of the stent, and the inward concave portion moves toward the axis of the stent. Please refer to Fig. 4e, the inward bent mesh cell has a bent portion referring to the portion of the inward bent mesh cells from the bending point 132 to the end 131. The profile line of the bent portion is an arc, a straight line, a broken line, or a combination thereof. Specifically, referring to Fig. 4a, the profile line of the bent portion resulting from the first end mesh cell 13' being inward bent toward the axis of the stent. Referring to Fig. 4b, the profile line of the bent portion resulting from the first end mesh cell 13' being inward bent toward the axis of the stent forms a broken line. Referring to Fig. 4c, the profile line of the bent portion resulting from the first end mesh cell 13' being inward bent toward the axis of the stent. In yet another embodiment, please refer to Fig. 4d, the profile line of the bent portion resulting from the first end mesh cell 13' being inward concave toward the axis of the stent forms a broken line, and the bent portion is a portion of the inward concave mesh cell from the bending point 132 to the end 131. When inward bending is adopted, the profile line of the bent portion resulting from the inward bending may be an arc, a straight line, a broken line or a combination thereof. An arc or a straight line is preferred, which allows a convenient production. In the plane defined by the profile line and the axis of the frame 1, the inward concave angle α and the inward bending angle β of the first end mesh 13' shall satisfy 0.1°≤α/β≤180°. Please refer to Fig. 4d, the concave angle α is the angle between the profile line 133 before concaving and the line connecting the bending point 132 of the mesh cell and the end 131 of the mesh cell. Please refer to Fig. 4e, inward bending angle β is the angle between the profile line 133 before the inward bending of the mesh cell and the line connecting the bending point 132 of the mesh cell and the end 131 of the mesh cell. The inward concave angle α and the inward bending angle β preferably satisfy 10° ≤α/β≤80°, which allows a convenient production.

Those skilled in the art is able to form the end mesh cell having a shorter projection in the axial direction by inward curling, bending, concaving or shortening the rods of the first end mesh cell 13', or a combination thereof. This embodiment does not specifically limit this.

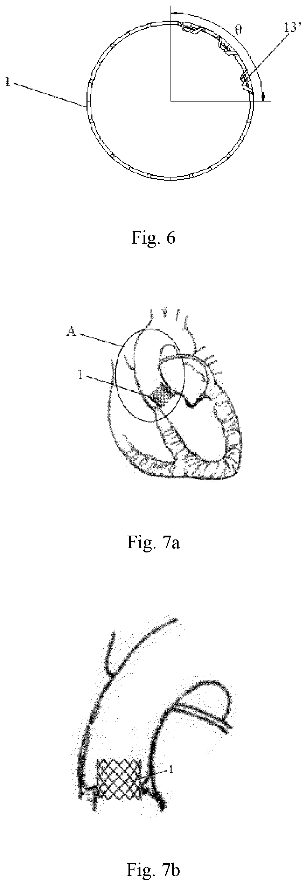

Fig. 6 shows an area where the first end mesh cells 13' are located in the circumferential direction. Taking the projection of the inflow tract of the cylindrical stent as an example, the distribution angle θ formed by the first end mesh cell 13' in the circumferential direction satisfies 20°≤θ≤120°, and preferably θ is 90°. After the valve stent is implanted, the area where the first end mesh cells 13' are located corresponds to the part of the cardiac anatomy where the conduction bundles are concentrated, so as to avoid the compression of the conduction bundles. It can be seen from Figs. 7a and 7b, the compression of the conduction bundles is significantly avoided, thereby reducing the probability of conduction block.

In the prior art, in order to avoid the compression of the conduction bundles, a gap is defined on the frame of the valve stent at the portion thereof positionally corresponding to the conduction bundles. As a result, the rods of the end mesh cells in positional correspondence with the conduction bundles are removed, and thus the mesh cells are not complete, which damages the radial support of the stent. Therefore, it is likely to cause a deformation and even a collapse of the stent. This design is not suitable for the aortic valve system because the blood flow pressure and cardiac pulsation pressure that the aortic valve stent needs to bear are significantly larger. The radial support required for the stent is thus greater. It is proved by test experiments that, compared with the traditional stent with incomplete mesh cells, the support performance of the frame of the valve stent provided by the present invention is significantly better.

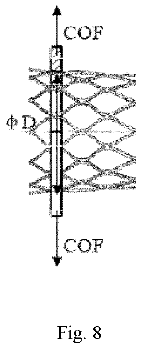

Referring to Fig. 8, which shows a measurement of the chronic outward force (COF) that the two stent frames can bear on the first node of the end mesh cells. The larger the value of COF is, the better the circumferential support will be, The "notched stent" represents the existing stent with a gap. The "inward curled stent" represents the stent proposed by the present invention. The experimental results show that the support performance of the valve stent provided by the present invention is significantly better than that of the stent with incomplete mesh cells.

Experimental method: the notch or inward curled portion of the tested stent is located at the end thereof; there are two orientations of the stent when tested, which are that the tested structure faces up and the test structure faces the tester (i.e., faces outward). The test results are as follows :

| Stent Type | Stent Type | Measuring Position | Mechanical Properties | Local COF - N | | | |
|---|---|---|---|---|---|---|---|
| | | | Current Size | Specifications: Φ17 | | | |
| | | | Compression | First time | Second time | Third time | Mean Value |
| Nitinol Stent | Notched Stent | First Node | Notch (up) | 13.39 | 13.22 | 13.38 | 13.33 |
| | | | Notch (outward) | 13.93 | 13.61 | 13.35 | 13.63 |
| | Inward Curled Stent | First Node | Inward Curled Portion (up) | 15.50 | 15.82 | 16.48 | 15.93 |
| | | | Inward Curled Portion (outward) | 16.44 | 16.32 | 16.54 | 16.43 |

It can be seen from the above table that the COF that the inward curled stent proposed by the present invention can withstand on the first node of the end mesh cells thereof is significantly greater than the COF that the existing notched stent can withstand on the first node of the end mesh cells thereof. Therefore, the support performance of the valve stent provided by the present invention is significantly better than that of the stent with incomplete mesh cells.

In conclusion, in the valve stent and the prosthesis thereof according to the present invention, the end of some of the end mesh cells are inward curled, bent, concave, or the rods of the end mesh cells are shortened, so as to be in positional correspondence with the conduction bundles of the native valve annulus. As a result, a height difference occurs in positional correspondence with the conduction bundles, which reduces the risk of conduction block. Compared with the traditional notched stent with incomplete mesh cells structure, the local radial support of the valve annulus is better, which can reduce the risk of conduction block while providing the radial support as required. When it is formed by inward curling, inward bending, or inward concave, the stent has a blunt end for contact with tissues, which further avoids the compression and contusion of the tissues as well as conduction bundles. In addition, the inward curled end of the end mesh cells makes the visualization area more concentrated or enlarged, which is more conducive to the operator's observation, accurate positioning, precise fine-tuning or retrieval of the valve stent.

Although the present invention has been disclosed with reference to the preferred embodiments, they are not intended to limit the invention in any sense. Any person skilled in the art may make modifications or improvements without departing from the spirit and scope of the invention. Accordingly, the scope of the invention shall be as defined by the appended claims.

## Claims

1. A valve stent, comprising a frame comprising a plurality of mesh cells connected with each other, wherein a plurality of end mesh cells are provided at two ends of the frame, at least one of the end mesh cells is a first end mesh cell, all of the end mesh cells at an end where the first end mesh cell is located are arranged continuously and completely in a circumferential direction, and a height of a projection of the first end mesh cell in an axial direction is lower than a height of a projection of rest of the end mesh cells at the end where the first end mesh cell is located.

2. The valve stent according to claim 1, wherein an end of the first end mesh cell is inward curled toward an axis of the valve stent to form an inward curled mesh cell

3. The valve stent according to claim 2, wherein in a plane defined by a profile line of the inward curled mesh cell and the axis of the valve stent, an inward curling angle γ satisfies 90°≤γ≤270°.

4. The valve stent according to claim 2, wherein a vertical distance L from a center of a curled portion of the inward curled mesh cell to a profile line is greater than or equal to an arc radius R of the curled portion, wherein the arc radius R is constant or variable.

5. The valve stent according to claim 1, wherein an end of the first end mesh cell is inward bent or concave toward an axis of the valve stent to form an inward bent mesh cell or an inward concave mesh cell, and the inward bent mesh cell has a bent portion having a profile line in a form of an arc, a straight line, a broken line, or a combination thereof.

6. The valve stent according to claim 5, in a plane defined by a profile line of the inward bent mesh cell or of the inward concave mesh cell and the axis of the valve stent, an inward concaving angle α and an inward bending angle β satisfy 10°≤α/β≤80°.

7. The valve stent according to claim 1, wherein the first end mesh cell is quadrilateral with four sides formed by rods, and two adjacent ones of the rods forming an end of the first end mesh cell is shorter than other two of the rods

8. The valve stent according to claim 1, wherein a height difference H in the axial direction between the projection of the first end mesh cell and the projection of the rest of the end mesh cells at the end where the first end mesh cell is located satisfies 0mm<H≤12mm.

9. The valve stent according to claim 8, wherein height difference H satisfies 2≤H≤4mm.

10. The valve stent according to claim 1, wherein an angle θ resulting from the circumferential arrangement of the first end mesh cell satisfies 20°≤θ≤120°.

11. A valve prosthesis, comprising the valve stent according to any one of claims 1-10 and a membrane, wherein the membrane is arranged in the frame of the valve stent.
